# EUROPEAN PATENT SPECIFICATION

(11) **EP 3 490 679 B1**
(45) Date of publication and mention of the grant of the patent: **09.09.2020**
(21) Application number: 17749162.8
(22) Date of filing: 28.07.2017
(51) Int. Cl.: A61Q 1/14, A61Q 19/02, A61K 8/86, A61B 18/20, A61Q 17/04, A61B 17/00, A61B 18/00

(54) **A PERFLUOROPOLYETHER FOR USE IN SKIN LIGHT THERAPY TREATMENT**
PERFLUORPOLYETHER ZUR VERWENDUNG IN DER HAUTLICHTTHERAPIEBEHANDLUNG
FLUOROPOLYÉTHER POUR UTILISATION DANS UN TRAITEMENT DE THÉRAPIE LUMINEUSE DE LA PEAU

(30) Priority: 29.07.2016 EP 16382375
(43) Date of publication of application: 05.06.2019
(73) Proprietor: Isdin, S. A., 08019 Barcelona (ES)
(72) Inventor: TRULLÀS CABANAS, Carles, 08019 Barcelona (ES); FOYACA FERRER, Monica, 08019 Barcelona (ES)
(74) Representative: ZBM Patents - Zea, Barlocci & Markvardsen
(86) International application number: PCT/EP2017/069177
(87) International publication number: WO 2018/020003

(56) References cited:
- EP-B1- 1 073 470
- WO-A1-02/03925
- WO-A1-2013/032535
- US-A- 5 578 311
- US-A- 5 902 569
- US-A- 6 074 652
- US-A1- 2010 145 256
- L. RIGANO ET AL: "Use and properties of perfluoropolymethyl-isopropylethers in skin and hair cleaning; system stabilization and interference with sebum redistribution on skin and hair", INTERNATIONAL JOURNAL OF COSMETIC SCIENCE., vol. 11, no. 6, 1 December 1989 (1989-12-01), pages 259-282, XP055323066, NL ISSN: 0142-5463, DOI: 10.1111/j.1467-2494.1989.tb00516.x
- MAHRHAUSER DENISE-SILVIA ET AL: "Double emulsions based on silicone-fluorocarbon-water and their skin penetration", INTERNATIONAL JOURNAL OF PHARMACEUTICS, ELSEVIER, AMSTERDAM, NL, vol. 498, no. 1, 11 December 2015 (2015-12-11), pages 130-133, XP029389357, ISSN: 0378-5173, DOI: 10.1016/J.IJPHARM.2015.12.023
- POKOTILOVSKI YU N ET AL: "The experimental and calculated density of states and UCN loss coefficients of perfluoropolyether oils at low temperatures", PHYSICA B: CONDENSED MATTER, ELSEVIER, AMSTERDAM, NL, vol. 403, no. 10-11, 1 May 2008 (2008-05-01), pages 1942-1948, XP022711818, ISSN: 0921-4526, DOI: 10.1016/J.PHYSB.2007.10.377 [retrieved on 2007-11-17]

## Description

This application claims the benefit of European Patent Application 16382375.0 filed July 29th 2016.

The present invention relates to the field of skin light therapy treatment, in particular, it relates to a perfluoropolyether for use in light therapy treatment of a skin disease, as well as the non-therapeutic use of the perfluoropolyether in a light therapy treatment of a skin condition, as well as to a composition containing the perfluoropolyether suitable for skin light therapy treatment.

### BACKGROUND ART

Light therapy or phototherapy, particularly laser therapy, is perphaps one of the best treatment regimen available to date for the treatment of a skin disease or condition. Physicians often use laser therapy involving lasers operating at a variety of wavelengths and power and fluence levels. Typically, Q-switched Nd:YAG lasers operating at 1064 nm, and Q-switched alexandrite lasers operating at 755 nm are commonly used. However, other type of lasers such as for example dye lasers operating in the visual portion of the spectrum, or frequency doubled Nd:YAG lasers operating at 532 nm (commonly called as "KTP lasers") are also used.

In the recent years, some problems and adverse events associated to skin phototherapies have been disclosed. Particularly, it is known that the exposure of a skin area to a light output can create conditions within the treatment area that tend to reduce the effectiveness of subsequent laser exposures. One of that conditions is the so called "whitening reaction".

The "whitening" or "whitening reaction" refers to any event or sequence of events that causes a negative therapeutic effect resulting from exposure of a treatment area to a light output. These events may include one or more chemical reactions and/or physical changes. One example of this negative effects of light therapies is that the laser induces immediate whitening reactions that are hypothesized to result from thermally induced cavitation bubble formation. It is disclosed that the presence of these bubbles of gas in the treatment area may attenuate or weak the delivery of light to the skin treatment area in the subsequent light passes becuase the light impings on surface of the bubbles and scatters in multiple directions, including away from the treatment area. Thus, bubbles presence reduces light therapy effectiveness.

As bubbles of gas fade over spontaneously about twenty minutes after the last light exposure, clinicians may avoid some of the adverse consequences of whitening by simply waiting for the naturally resolution of the unwanted whitening. However, the fact of waiting the spontaneously facing over of bubbles implies lengthy treatment times, that often poses significant problems for patients and clinicians because multiple treatment sessions are required over a period of months to achieve desired results.

Thus, certain methods and systems for the resolution of whitening reaction have been proposed in the state of the art having the aim of resolving the whitening reaction by controlling, reducing, minimizing or eliminating the generation of these bubbles of gas.

Particularly, the PCT patent application WO2013032535 discloses the use of perfluorodecalin as a chemical facilitator to resolve post-laser whitening in phototherapy treatment in tattoo removal. In particular, it specifically discloses an occlusive method for tattoo removal that implies applying several coats of perfluorodecalin on the skin treatment area with a cotton-tipped applicator followed by covering the treated area with a transparent dressing or patch; and then exposing the covered tattoo to laser outputs. However, the use of a patch soaked with the perfluorodecalin is disadvantageous because it is practicaly impossible to dose the effective amount of the chemical facilitator and further, the physical and physiological features of the patch or dressing such as its transparence can hinder the resolution of the whitening reaction. Furthermore, another disadvantage is that the commonly used patch soaked with the perfluorodecalin do not cover the whole skin surface to be treated (that is the whole surface of the tattoo) and therefore, the physician has to move the patch during the light therapy.

Furthermore, the US5578311 discloses cosmetic compositions, such as sunscreen or skin protector, comprising (A) a fluorine compound-treated power, (B) from 1-70% by weight of perfluoroorganic compund such as perfluorodecalin or perfluoropolyether such as Fomblin, and (C) a silicone oil. In particular, the compositions give an appropriate moist and refreshing feel after its application on the skin. Meanwhile, the US6074652 discloses foundatoins comprising 1-2% by weight of perfluoropolymethylisopropyl ether and decamethylcyclopentasiloxane. Finally, in the US5902569 it is disclosed UV shielding compositions, especially a foundation comprising 8% of Fomblin HC-04 and methyl polysiloxane. It is also disclosed that the compositions provide moisture with smooth skin texture.

Therefore, from what is known in the art it is derived that there is still the need of providing a more effective method for resolving whitening in a skin light therapy treatment.

### SUMMARY OF THE INVENTION

Inventors have found that the use of a perfluoropolyether in a light therapy treatment of a skin desease or condition allows a greater resolution of whitening, specially in the removal of tattoos. In particular, the topical application of a perfluoropolyether on the skin treatment area prior to the exposure of a light output allows reducing the generation of bubbles.

It is advantageous because the topical application of perfluoropolyether in skin light therapy allows resolving the post-laser whitening in a much shorter period of time than the chemical facilitator previously disclosed in the state of the art. Furthermore, the use of a perfluoropolyether also increases the effectivity of each light pass and permits increase the number of laser passes per each session and also reducing the number of sessions required to achieve for instance the removal of a tattoo.

Additionally, the inventors have also found that the use of a perfluoropolyether in skin light therapy treatments also improve the healing of the skin treated area; and further, patients subjected to the light therapy treatment of the present invention have a higher reduction of burning, scarring, pain, hypopigmentation, hyperpigmentation, blistering, oozing and crusting.

Finally, the perfluoropolyether is also advantageous because allows preparing topical compositions containing the appropriate effective amount of the active ingredient, and further the above mentioned topical compositions which comprises the effective amount of the perfluoropolyether are capable of forming a transparent film suitable for delivering a light output through it.

Thus, the invention refers to a non-therapeutic use of a perfluoropolyether in a light therapy treatment of a skin condition, wherein the skin condition is skin with tattoo, wherein the ligth therapy treatment comprises: (a) first applying a topical composition comprising an effective amount of the perfluoropolyether together with one or more topically acceptable excipients or carriers to the skin treatment area; and then (b) exposing the skin treatment area of step (a) to one or more light outputs. It means that the perfluoropolyether is applied to a subject having a skin condition suffering from skin dyspigmentation, benign pigmented lesions and skin with tattoo.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

For purposes of the present invention, any ranges given include both the lower and the upper end-points of the range. Ranges given, such as temperatures, times, and the like, should be considered approximate, unless specifically stated.

As it was disclosed above an aspect of the invention is a perfluoropolyether for topical use to resolve whitening in light therapy treatment. The term "resolve" whitening refers to the control, reduction, speeding, minimizing or elimination of any event or sequence of events that causes a negative therapeutic effect resulting from exposure of a treatment area to a light output, for instance the generation of bubbles of gas generated by a light exposure.

The term "transparent" film refers to a film that permits visible light to pass through so that bodies situated behind are seen clearly.

The term "perfluoropolyether" refers to a polyether in which all of the hydrogens on all of the hydrocarbon groups are replaced with fluorine atoms. Further, the term "ether group" is used herein to refer to any oxygen atom between two carbon atoms; and the term "polyether" refers to a molecule having more than one ether group, preferably at least two ether groups separated by aliphatic or aromatic carbon atoms or combination thereof.

In an embodiment, optionally in combination with one or more features of the various embodiments described above or below, the perfluoropolyether is a compound of formula (I) 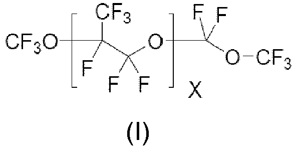 wherein x is an integer comprised from 10 to 60; preferably comprised from 20 to 40. In a particular embodiment, optionally in combination with one or more features of the various embodiments described above or below, the perfluoropolyether is a compound of formula (I) wherein x is 30.

As it is disclosed above, the perfluoropolyether is useful prior a light therapy treatment. The term "light therapy" refers to the use of one or more light sources of any type that emits light (i.e. an emitter light outputs) with a wavelength between about 400 nm and 10600 nm. The light therapy treatment occurs by the use of an emitter or light-generating device such as for example a laser; a lamp for instance a flash lamp (as used in an intense pulsed light); or a light output device. In an embodiment, optionally in combination with one or more features of the various embodiments described above or below, the light therapy treatment is a laser therapy treatment. The term "laser therapy" refers to the use of lasers that emit specific wavelengths within the UV, visible and IR portions of the electromagnetic spectrum. Examples of lasers commonly used include, but it is not limited to Q-switched Nd:YAG laser, Q-switched ruby laser, Q-switched Alexandrite laser. Typically, laser therapy treatments can be performed mantaining the fluence of laser during the entire treatment, or alternatively modifying the fluence from a relative low fluence to a high fluence. In an embodiment, optionally in combination with one or more features of the various embodiments described above or below, the light therapy is a laser therapy, wherein the treatment is performed mantaining the fleunce of the laser dring the entire tretament.

In general, a light application may include one or more exposures of a treatment area of one or more emitter light outputs. In those circumstances in which an emitter output covers only a portion of an intended treatment area, multiple outputs may be necessary to obtain full treatment. That is, if an emitter output for instance appears relatively small and circular in shape at the surface of the treatment area, clinicians or others may use two or more overlapping outputs, which may be fully or partially overlapping, to treat a larger and/or non-circular treatment area. Typically, a treatment area may receive or be exposed to sufficient outputs in such manner as to achieve the desired result.

In an embodiment, optionally in combination with one or more features of the various embodiments described above or below, the skin treatment area is exposed to a number of light outputs comprised from one to 5; preferably comprised from two to 5. In a particular embodiment, optionally in combination with one or more features of the various embodiments described above or below, the skin treatment area is exposed to 2, 3, 4 or 5 light outputs; preferably to 2-3 light outputs.

For convenience, the term "treatment session" refers to a one single or group of "treatment passes" that occurs together in a single time period. Particularly, each treatment pass may be separated by minuts. Meanwhile, a certain number of "treatment sessions" may be identified based upon the amount of time between two or more groups of "treatment passes", typically a period from days to months are needed between each treatment session to achieve the completed treatment of the skin disease or condition. In an embodiment, optionally in combination with one or more features of the various embodiments described above or below, wherein when the skin treatment area is exposed to a number of outputs comprised from 1 to 5; preferably from 2 to 5, then the time interval between the light treatment passes is less than 10 minutes; preferably less than 5 min; more preferably less than 1 minute. In an embodiment, optionally in combination with one or more features of the various embodiments described above or below, wherein when the skin treatment area is exposed to a number of outputs comprised from 1 to 5; preferably comprised from 2 to 5, then the time interval between the light treatment passes is less than 1 minute. The method of the invention is advantageous because less additional applications of the perfluoropolyether prior the subsequent laser exposures is needed to achieve the complete treatment. Therefore, the total amount of perfluoropolyether used per treatment session is significantly reduced.

In an embodiment, optionally in combination with one or more features of the various embodiments described above or below, the application of the perfluoropolyether is performed without skin occlusion. The application of the composition of perfluoropolyether on the skin treatment area allows the formation of an appropriate film for being used in light therapy treatment without compromising the resolution of the light applied.

In the present disclosure the perfluoropolyether is suitable for the topical use in light therapy treatments of skin diseases that may benefit from the exposure of a more effective light output, wherein the skin disease is selected from the group consisiting of skin dyspigmentation, varicosities, a vascular lesion, telangiectasis and benign pigmented lesion.

In an embodiment, optionally in combination with one or more features of the various embodiments described above or below, the perfluoropolyether is suitable for the topical non-therapeutic use in light therapy treatment of a skin condition that may benefit from the exposure of a more effective light output, wherein the skin condition is skin with a tattoo. The use of the perfluoropolyether for the tattoo removal is especially advantageous because allows a higher removal of tattoo in each laser pass. Thus, it is also advantageous because allows increasing the light passes per session and also reducing the number of sessions required to achieve the removal of the tattoo.

In step (a) of the light therapy treatment as defined in the present invention, the perfluoropolyether is applied on the treatment area in form of a composition comprising an effective amount of the perfluropolyether together with one or more topically acceptable excipients or carriers. The term "effective amount" of the perfluoropolyether refers to the amount of the active ingredient which provides the effect of resolving the whitening reaction associated to light therapy treatment. In an embodiment, optionally in combination with one or more features of the various embodiments described above or below, the effective amount of the perfluoropolyether is comprised from 0.1 % to 10 % by weight of the total weight of the composition; preferably comprised from 0.1 % to 5% by weight of the total weight of the composition; more preferably comprised from 0.5% to 5% by weight of the total weight of the composition. In a particular embodiment, the effective amount of the perfluoropolyether is comprised from 1 % to 5 % by weight of the total weight of the composition; preferably comprised from 1.5% to 5% by weight of the total weight of the composition. In a particular embodiment, optionally in combination with one or more features of the various embodiments described above or below, the effective amount of the perfluoropolyether is 1.5% by weight of the total weight of the composition. The term "percentage (%) by weight" refers to the percentage of each ingredient of the composition in relation to the total weight of the composition.

The compositions as defined above comprise appropriate excipients or carriers for topical administration that includes, but not limited to, repairing cutaneous barrier function agent, a hydrating agent, an emollient, an emulsifier, a surfactant, a thickener, a chelant, a humectant, a pH-regulating agent, an antioxidant, a preservative agent, a skin conditioning, a vehicle, or their mixtures. Thus, the terms "topically acceptable" or "dermatological acceptable" have the same meaning and are used interchangeable. They refer to that excipients or carriers suitable for use in contact with human skin without undue toxicity, incompatibility, instability, allergic response, among others, and used in an amount adequate to provide the desired consistency, and ease application.

The term "emollient" agent refers to a material that softens and soothes the skin in order to correct dryness and scaling of the skin, lubricating the skin surface, encouraging skin water retention, and altering product textures. Examples of appropriate topical emollient agents include, but are not limited to, octyl hydroxystearate, lanolin, caprylic/capric triglyceride, cetyl palmitate, octyldodecanol, cetyl alcohol, isopropyl isostearate, glyceryl dilaurate, isopropyl myristate, palm alcohol, dimethicone, squalane, plukenetia volubilis seed oil, butyrospermum parkii butter, sucrose cocoate, or their mixtures. Preferably the emollient is selected from the group consisting of dimethicone, squalane, plukenetia volubilis seed oil, butyrospermum parkii butter, caprylic/capric triglyceride, octyldodecanol, or their mixtures. The amount of emollient agent in the compositions of the present invention is comprised from 8% and 25% by weight.

The term "emulsifying agent" or "emulsifier" which is herein used interchangeably refers to a material that reduces surface tension, promoting the formation of intimate mixtures of non-miscible liquids by altering the interfacial tension. Emulsifier stabilizes an emulsion by increasing its kinetic stability. Examples of appropriate emulsifier include, but are not limited to, glyceryl trioleate, glyceryl oleate, acetylated sucrose distearate, sorbitan trioleate, polyoxyethylene monostearate, glycerol monooleate, sucrose distearate, polyethylene glycol monostearate, octyl phenoxypoly (ethyleneoxy) ethanol, deacylerin penta-isostearate, sorbitan sesquioleate, hydroxylated lanolin, lecithin, lanolin, triglyceryl diisostearate, polyoxyethylene oleyl ether, calcium stearoyl-2-lactylate, sodium lauroyl lactylate, sodium stearoyl lactylate, cetearyl glucoside, methyl glucoside sesquistearate, sorbitan monopalmitate, methoxy polyethylene glycol-22/dodecyl glycol copolymer,PEG-8 beeswax, PEG-6 isostearate, polyethylene glycol-45/dodecyl glycol copolymer, polyethylene glycol 400 distearate and glyceryl stearate, carbomer, candelilla/jojoba/rice bran polyglyceryl-3 esters, cetyl phosphate, potassium cetyl phosphate, or their mixtures. Preferably, the emulsifier is selected group consisting of glyceryl oleate, lecithin, sodium lauroyl lactylate, sodium stearoyl lactylate, glyceryl stearate, PEG-8 beeswax, PEG-6 isostearate, carbomer, candelilla/jojoba/rice bran polyglyceryl-3 esters, or their mixtures. The amount of the emulsifier in the compositions of the present invention is comprised from 1% and 10% by weight.

The term "surfactant" refers to a material which lowers the surface tension of a liquid and the interfacial tension between two liquids, allowing their easier spreading. Surfactants have a hydrophilic head that is attracted to water molecules and a hydrophobic tail that repels water and simultaneously attaches itself to oil and grease in dirt. These opposing forces loosen the dirt and suspend it in the water, having the ability to remove it from surfaces such as the human skin, textiles, and other solids, when surfactants are dissolved in water. Examples of appropriate surfactant agents include, but are not limited to, non-ionic, ionic (either anionic or cationic) or zwitterionic (or amphoteric wherein the head of the surfactant contains two oppositely charged groups) surfactants. Examples of anionic surfactants include, but are not limited to, those based on sulfate, sulfonate or carboxylate anions such as perfluorooctanoate (PFOA or PFO), alkyl benzene sulfonate, soaps, fatty acid salts, or alkyl sulfate salts such as perfluorooctanesulfonate (PFOS), sodium dodecyl sulfate (SDS), ammonium lauryl sulfate, or sodium lauryl ether sulfate (SLES). Examples of cationic surfactants include, but are not limited to, those based on quaternary ammonium cations such as or alkyltrimethylammonium including cetyl trimethylammonium bromide (CTAB) a.k.a., or hexadecyl trimethyl ammonium bromide, cetylpyridinium chloride (CPC), polyethoxylated tallow amine (POEA), benzalkonium chloride (BAC), or benzethonium chloride (BZT). Examples of zwitterionic surfactants include, but are not limited to dodecyl betaine, cocamidopropyl betaine, or coco ampho glycinate. Examples of non-ionic surfactants include, but are not limited to, alkyl poly(ethylene oxide), alkylphenol poly(ethylene oxide), copolymers of poly(ethylene oxide), poly(propylene oxide) (commercially called Poloxamers or Poloxamines), alkyl polyglucosides including octyl glucoside and decyl maltoside, fatty alcohols including cetyl alcohol and oleyl alcohol, cocamide MEA, cocamide DEA, or polysorbates including tween 20, tween 80, PEG-8 or dodecyl dimethylamine oxide. Preferably, the surfactant is skin friendly, including polyethylene glycol, or their mixtures. The amount of the surfactant in the compositions of the present invention is comprised from 0,01% and 5% by weight.

The term "pH-regulating" agent refers to acids or bases that can be used to adjust the pH of the finished product to the desired level, without affecting the stability of the solution. Examples of appropriate topical pH-regulating agents include, but are not limited to, acetic acid, lactic acid, citric acid, ethanolamine, formic acid, oxalic acid, potassium hydroxide, sodium hydroxide, triethanolamine, or their mixtures. Preferably, the pH-regulating agent is selected group consisting of triethanolamine, sodium hydroxide, lactic acid, citric acid and mixture thereof. The amount of the pH-regulating agent in the compositions of the present invention is comprised from 0,05% % and 1% by weight.

The term "preservative" refers to a material that prevents or reduces or slows down microbial growth, providing that the stability of the solution is not affected. Examples of appropriate preservative agents include, but are not limited to, benzoic acid, butylparaben, ethylparaben, propylparaben, methylparaben, sorbic acid, potassium sorbate, sodium benzoate, phenoxyethanol, triclosan, or their mixtures. Preferably, the preservative agent is selected group consisting of potassium sorbate, sodium benzoate, phenoxyethanol, and mixture thereof.

The terms "chelant", "chelating agent", "sequestering agent" and "complexing agent" refers to a compound capable of solubilizing or complexing metal ions, or the ability of a compound to lower hardness in a given paint spray booth system. Examples of appropriate chelant include, but are not limited to, ethylenediaminetetraacetic acid (EDTA), nitrilotriacetic acid (NTA), diethylenetriamine pentacetic acid (DTPA), N-hydroxyethylethylene diaminetriacetic acid (HEDTA), tetraborates and triethylamine diamine, and mixture, salts and derivatives thereof. The amount of the chelant in the compositions of the present invention is comprised from 0,001% and 0.2% by weight.

The term "antioxidant" refers to a material that slows or prevents the oxidation of other molecules. Antioxidants include free radical scavengers and reducing agents. Examples of appropriate antioxidants include, but are not limited to, free radical scavengers or reducing agents such as, acetyl cysteine, ascorbic acid, ascorbyl palmitate, butylated hydroxytoluene, green tea extract, caffeic acid, cysteine, tocopherol, ubiquinone, propyl gallate, butylated hydroxytoluene (BHT), and their mixtures. Preferably, the antioxidant agent is selected group consisting of ascorbyl palmitate, tocopherol, and mixture thereof. The amount of the antioxidant in the compositions of the present invention is comprised from 0.002% and 0.5% by weight.

The term "skin conditioning" refers to an agent the maintain the human skin in a good condition, including regulating the skin condition, preventing or reducing visible and/or tactile discontinuities in skin, and/or protecting the skin. The term "regulating skin condition" includes delaying, minimizing and/or preventing visible and/or tactile discontinuities in skin; ameliorating, e.g., diminishing, minimizing and/or effacing, discontinuities in skin; as well as improving skin appearance and/or feel.

The compositions mentioned above also include a vehicle. Examples of vehicles include, but are not limited to, water, propylene glycol, butylene glycol, ethanol, isopropanol, silicones or mixture thereof. Preferably, the vehicle is water.

Additionally, the compositions of the present invention may contain other ingredients, such as fragrances, colorants, and other components known in the state of the art for use in topical formulations.

The term "excipient or carrier capable of forming a transparent film in contact with skin" refers to a film-forming material that allows the formation of a transparent film in contact with the skin appropriate for being used in light therapy treatments. Examples of "excipients or carriers capable of forming a transparent film in contact with skin" include, but are not limited to, silicone or a derivative thereof; and polivinylpyrrolidone. Preferably, the excipient or carrier capable of forming a transparent film in contact with skin is a silicone or a derivative thereof; particularly the silicone is dimethicone.

The topical compositions of the invention can be formulated in several forms that include, but are not limited to, solutions, creams, mousses, lotions and gels. In an embodiment, optionally in combination with one or more features of the various embodiments described above or below, the topical composition used is formulated preferably as an emulsion. An emulsion is a dispersed system comprising at least two immiscible phases, one phase dispersed in the other as droplets. The above mentioned emulsifying agents are included to improve stability. When water is the dispersed phase and oil is the dispersion medium, the emulsion is termed a water-in-oil emulsion (w/o). When oil is dispersed as droplets throughout the aqueous phase, the emulsion is termed an oil-in-water emulsion (o/w). Other types of emulsions known in the art are multiple emulsions, such as water-in-oil-in-water emulsions (w/o/w), GELTRAP emulsions, where the aqueous intern phase is gelified and it is covered by the oil phase, and SWOP emulsions, also known as inversion emulsions. The emulsions used are preferably oil-in-water emulsions. In a preferred embodiment, optionally in combination with one or more features of the various embodiments described above or below, the topical compositions of the invention is in form of creams and lotions; more preferably in form of creams.

Topical compositions of the present invention can be prepared according to methods well known in the state of the art. The appropriate excipients and/or carriers, and their amounts, can readily be determined by those skilled in the art according to the type of formulation being prepared.

As it is mentioned above, the topical compositions of the present invention which comprises an appropriate effective amount of the perfluoropolyether are capable of forming a transparent film on the skin suitable for delivering a light output through it. Furthermore, the compositions of the present invention are advantageous because allows reducing the amount of active ingredient applied on the skin in each session.

In en embodiment, optionally in combination with one or more features of the various embodiments described above or below, the perfluoropolyether is applied on the treatment area in form of a composition comprising an effective amount of the perfluroether together with one or more topically acceptable excipients or carriers which comprises: from 0,1% to 10% by weight of the perfluoropolyether; from 8% to 15% by weight of at least one emollient; from 1 to 9% by weight of at least one emulsifying agent; from 0,01% to 3% by weight of at least one surfactant; from 0,05% to 1% by weight of at least one pH-regulating agent; from 0,001 to 0,12% by weight of at least one chelant; from 0,002% to 0,15% by weight of at least one antioxidant; from 0% % to 1% by weight of at least one preservative; from 0% to 5% by weight of at least one skin conditioning; and sufficient quantity of at least one solvent for being the sum of the ingredients 100%.

In an embodiment, optionally in combination with one or more features of the various embodiments described above or below, the perfluoropolyether is applied on the treatment area in form of a composition comprising an effective amount of the perfluroether together with one or more topically acceptable excipients or carriers which comprises: from 0,1 % to 5% by weight of the perfluoropolyether; from 8% to 10% by weight of at least one emollient; from 1% to 8% by weight of at least one emulsifying agent; from 0,01% to 0,05% by weight of at least one surfactant; from 0,05 to 0,95% by weight of at least one preservative; from 0,001% to 0,5% by weight of at least one pH-regulating agent; from 0,002% to 0,1% by weight of at least one chelant; from 0,003% to 0.03% by weight of at least one antioxidant; from 0,003% to 1% by weight of at least one skin conditioning; and sufficient quantity of at least one solvent for being the sum of the ingredients 100%.

In an embodiment, optionally in combination with one or more features of the various embodiments described above or below, the perfluoropolyether is applied on the treatment area in form of a composition comprising an effective amount of the perfluroether together with one or more topically acceptable excipients or carriers which comprises: about 1.5% by weight of the perfluoropolyether; about 10% by weight of at least one emollient; about 7% by weight of at least one emulsifying agent; about 0.04% by weight of at least one surfactant; about 0.015% by weight of at least one antioxidant; about 0.2% by weight of at least one pH-regulating agent; about 0.1% by weight of at least one chelant; about 1% by weight of at least one preservative; about 0.005% by weight of at least one skin conditioning; and sufficient quantity of at least one solvent for being the sum of the ingredients 100%.

As it was mentioned above, an aspect of the present disclosure relates to a perfluoropolyether for use in a light therapy treatment of a skin disease, wherein the skin disease is selected from the group consisting of skin dyspigmentation, varicosities, vascular lesions, telangiectasis, and benign pigmented lesions; wherein the ligth therapy treatment comprises: (a) first applying a topical composition comprising an effective amount of the perfluoropolyether together with one or more topically acceptable excipients or carriers to the skin treatment area; and then (b) exposing the skin treatment area of step (a) to one or more light outputs. This aspect could be also formulated as the use of the perfluoropolyether as defined above for the preparation of a medicament for a light therapy treatment of a skin disease, wherein the skin disease is selected from the group consisting of skin dyspigmentation, varicosities, vascular lesions, telangiectasis, and benign pigmented lesions; and the treatment comprises: (a) first applying a topical composition comprising an effective amount of the perfluoropolyether together with one or more topically acceptable excipients or carriers to the skin treatment area; and (b) then exposing the skin treatment area of step (a) to one or more light outputs. It also relates to a method for the topical light therapy treatment of a mammal suffering or is susceptible to suffer from a skin disease selected from the group consisting of skin dyspigmentation, varicosities, vascular lesions, telangiectasis, and benign pigmented lesions, wherein the method comprises: (a) first applying a topical composition comprising an effective amount of the perfluoropolyether together with one or more topically acceptable excipients or carriers to the skin treatment area; and then (b) exposing the skin treatment area of step (a) to one or more light outputs.

As it was mentioned above, the invention relates to a non-therapeutic use of a perfluoropolyether in a light therapy treatment of a skin condition, wherein the skin condition is skin with tattoo, wherein the ligth therapy treatment comprises: (a) first applying a topical composition comprising an effective amount of the perfluoropolyether together with one or more topically acceptable excipients or carriers to the skin treatment area; and then (b) exposing the skin treatment area of step (a) to one or more light outputs. This aspect could be also formulated as a non-therapeutical method for the light therapy treatment of a mamal suffering or susceptible to suffer from a skin skin with tattoo, wherein the treatment comprises: (a) first applying a topical composition comprising an effective amount of the perfluoropolyether together with one or more topically acceptable excipients or carriers to the skin treatment area; and then (b) exposing the skin treatment area of step (a) to one or more light outputs.

A further aspect of the disclosure relates to a topical composition comprising an effective amount of the perfluoropolyether together with one or more topically acceptable excipients or carriers as defined in the first aspect of the invention, wherein at least one of the excipients or carriers is capable of forming a transparent film in contact with skin. All the embodiments disclosed above for the perfluoropolyether for use of the invention applies also for the topical composition of this aspect of the disclosure.

It is also described in the disclosure the use of the perfluoropolyether for resolving whitening in skin cosmetic light therapy treatment to improve its appearance or to beautify, preserve, cleanse and protect it.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention.

### EXAMPLES

### General consideration

### 1. Topical composition

### 1.1. Compositions

The topical composition of example 1 illustrates an emulsion composition of the present invention, which comprises a perfluoropolyether of formula (I) wherein x is 30. Table 1 illustrates the quantitative topical composition of Example 1, where the amount of the ingredients is expressed in % by weight of the ingredient in relation to the total weight of the composition.

**Table 1**

| **Ingredients** | | | | | **Example 1** |
|---|---|---|---|---|---|
| **Phase** | **N°** | **INCI NAME** | | **Function** | **% (w/w)** |
| Phase A | 01 | Isostearyl isostearte | | Emollient | 8 |
| | 02 | PEG-8 beeswax | | Emulsifying agent | 6 |
| | 03 | Dimethicone | | Emollient | 2 |
| | 04 | PEG-6 isostearate | | Emulsifying agent | 1 |
| | 05 | Tocopherol | | Antioxidant | 0.01 |
| | 06 | Ascorbic acid | | Antioxidant | 0.0005 |
| | 07 | Ascorbyl palmitate | | Antioxidant | 0.0025 |
| Phase B | 08 | Aqua | | Solvent | q.s.p* 100 |
| | 09 | Phenoxyethanol | | Preservative | 0.91 |
| | 10 | Carbomer^{(a)} | | emulsifying agent | 0.6 |
| | 11 | Disodium EDTA | | Chelant | 0.1 |
| | 12 | PEG-8 | | Surfactant | 0.04 |
| | 13 | Tropolone | | Skin conditioning | 0.0046 |
| | 14 | **Polyperfluoromethyl-isopropyl ether** | | Active ingredient | 1.5 |
| | 15 | Citric acid | | pH-regulator | 0.0005 |
| Phase C | 16 | Sodium hydroxide | | pH-regulator | 0.20 |
| | | | Total weight | | 100 |

| | | | | | |
|---|---|---|---|---|---|
| ^{(a)} Carbomer is the generic name of the compound poly(acrylic acid) (or PAA) of CAS number 9003-01-4. * "q.s.p" means "as needed for". | | | | | |

### 1.2. Preparation process

The topical composition of Example 1 of the present invention was prepared following the process as defined below:

### A. Preparation of Phases:

### Phase A:

In a reactor preheated at 70-80°C components (01), (02), (03), (04), (05), (06), and (07) are added. The stirring is maintained until all components are totally dissolved.

### Phase B:

In other reactor preheated at 70-80°C components (08), (09), (10), (11), (12), (13), and (14) are added. The stirring is maintained until all components are totally dissolved.

### B. Process for manufacturing the emulsion:

To the above-obtained phase (A), the above-obtained phase (B) is added with stirring. Then, the emulsion thus obtained is stirred for 10 minutes. After that time, the resulting emulsion was cooled until room temperature was reached; and then, to the resulting emulsion, phase (C) is added in quantity sufficient to obtain a final pH 6-7 followed by homogenizing

### 2. Light therapy treatment

### 2.1. Tattoo removal

A tattoo was removed by laser treatment which comprises:
(a) applying the composition of Example 1 to the surface tattoo area and
(b) exposing the surface of the tattoo area obtained in step (a) to at least one output of a laser (for instance Alejandrita laser) without modification of the fluence of the laser output.

During and after the treatment, extent and duration of whitening reaction was assessed as well as appearance of adverse effects, including reduction of burning, scarring, pain, hypopigmentation, hyperpigmentation, blistering, oozing and crusting . Each patient was seen at 3-6 weeks for follow-up and possible continued treatment.

### 3. Results

### 3A. Whitening-Effectivenes of the light treatment

Immediate whitening after each laser pass was observed. However, the whitening reaction was resolved after 3-5 seconds from the previous output. Further, it was observed that the light treatment clear tattoo more rapidly.

Thus, it was concluded that the topical aplication of perfluoropolyether in light therapy treatment resolves post-laser whitening in a short period of time, permitting an increase of the number of laser passes per session, and a reduction of the number of sessions required to achieve the totally or practically total removal of the tattoo.

### 3B. Effect over other adverse effects associated to the light treatment

Patient subjected to the light therapy treatment for tattoo removal of the present invention improved healing of the treated skin area and also reduced the amount of burning, scarring, pain, hypopigmentation, hyperpigmentation, blistering, oozing and crusting.

In conclusion, the treatment was well-tolerated with a lower personal assessment of pain.

### REFERENCES CITED IN THE APPLICATION

1. PCT patent application WO2013032535

## Claims

1. A non-therapeutic use of a perfluoropolyether in a light therapy treatment of a skin condition, wherein the skin condition is skin with tattoo, wherein the ligth therapy treatment comprises:
(a) first applying a topical composition comprising an effective amount of the perfluoropolyether together with one or more topically acceptable excipients or carriers to the skin treatment area; and then
(b) exposing the skin treatment area of step (a) to one or more light outputs;

2. The non-therapeutic use of a perfluoropolyether according to claim 1, wherein the number of light outputs is comprised from 1 to 5.

3. The non-therapeutic use of a perfluoropolyether according to any of the claims 1-2, wherein when the number of outputs is comprised from 2 to 5, then the interval between the light outputs is equal to or less than 10 minutes.

4. The non-therapeutic use of a perfluoropolyether according to any of the claims 1-3, wherein the application of perfluoropolyether of step (a) is performed without skin occlusion.

5. The non-therapeutic use of a perfluoropolyether according to any of the claims 1-4, wherein the perfluoropolyether is a compound of formula (I) 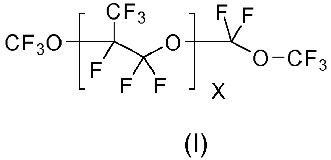 wherein x is an integer from 10 to 60.

6. The non-therapeutic use of a perfluoropolyether according to claim 5, wherein x is an integer from 20 to 40.

7. The non-therapeutic use of a perfluoropolyether according to any of the claims 5-6, wherein x is 30.

8. The non-therapeutic use of a perfluoropolyether according to any of the claims 1-7, wherein the light therapy treatment is a laser therapy treatment.

9. The non-therapeutic use of a perfluoropolyether according to any of the claims 1-8, wherein the topical composition comprises an effective amount of the perfluoropolyether comprised from 0.1% to 10% by weight of the total weight of the composition.

10. The non-therapeutic use of a perfluoropolyether according to any of the claims 1-9, wherein the topical composition comprises an effective amount of the perfluoropolyether comprised from 1.5% to 5% by weight of the total weight of the composition.

11. The non-therapeutic use of a perfluoropolyether according to any of the claims 1-10, wherein the topical composition comprises:
from 0,1% to 10% by weight of the perfluoropolyether;
from 8% to 15% by weight of at least one emollient;
from 1 to 9% by weight of at least one emulsifying agent;
from 0,01% to 3% by weight of at least one surfactant;
from 0,05% to 1% by weight of at least one pH-regulating agent;
from 0,001 to 0,12% by weight of at least one chelant;
from 0,002% to 0,15% by weight of at least one antioxidant;
from 0% % to 1% by weight of at least one preservative;
from 0% to 5% by weight of at least one skin conditioning; and sufficient quantity of at least one solvent for being the sum of the ingredients 100%.

12. The non-therapeutic use of a perfluoropolyether according to any of the claims 1-11, wherein the topical composition comprises:
from 0,1% to 5% by weight of the perfluoropolyether;
from 8% to 10% by weight of at least one emollient;
from 1% to 8% by weight of at least one emulsifying agent;
from 0,01% to 0,05% by weight of at least one surfactant;
from 0,05 to 0,95% by weight of at least one preservative;
from 0,001% to 0,5% by weight of at least one pH-regulating agent;
from 0,002% to 0,1% by weight of at least one chelant;
from 0,003% to 0.03% by weight of at least one antioxidant;
from 0,003% to 1% by weight of at least one skin conditioning; and
sufficient quantity of at least one solvent for being the sum of the ingredients 100%.

13. The non-therapeutic use of a perfluoropolyether according to any of the claims 1-12, wherein the topical composition comprises:
about 1.5% by weight of the perfluoropolyether;
about 10% by weight of at least one emollient;
about 7% by weight of at least one emulsifying agent;
about 0.04% by weight of at least one surfactant;
about 0.015% by weight of at least one antioxidant;
about 0.2% by weight of at least one pH-regulating agent;
about 0.1 % by weight of at least one chelant;
about 1% by weight of at least one preservative;
about 0.005% by weight of at least one skin conditioning; and
sufficient quantity of at least one solvent for being the sum of the ingredients 100%.

## Patentansprüche

1. Eine nicht-therapeutische Verwendung von einem Perfluoropolyether in einer Lichttherapiebehandlung von einer Hautkondition, wobei die Hautkondition tätowierte Haut ist, wobei die Lichttherapiebehandlung folgendes umfasst:
(a) zunächst die Anwendung von einer topischen Zusammensetzung umfassend: eine wirksame Menge des Perfluoropolyethers zusammen mit einem oder mehreren topisch akzeptablen Hilfsstoffen oder Trägersubstanzen auf die Hautbehandlungsfläche; und dann
(b) die Aussetzung der Hautbehandlungsfläche des Schritts (a) einem oder mehreren Lichtausgängen;

2. Die nicht-therapeutische Verwendung von einem Perfluoropolyether nach Anspruch 1, wobei die Anzahl von Lichtausgängen von 1 bis 5 reicht.

3. Die nicht-therapeutische Verwendung von einem Perfluoropolyether nach einem der Ansprüche 1-2, wobei, wenn die Anzahl an Ausgängen von 2 bis 5 reicht, die Zeitspanne zwischen den Lichtausgängen 10 Minuten oder weniger beträgt.

4. Die nicht-therapeutische Verwendung von einem Perfluoropolyether nach einem der Ansprüche 1-3, wobei die Anwendung von Perfluoropolyether des Schritts (a) ohne Okklusion der Haut durchgeführt wird.

5. Die nicht-therapeutische Verwendung von einem Perfluoropolyether nach einem der Ansprüche 1-4, wobei das Perfluoropolyether eine Verbindung der Formel (I) ist 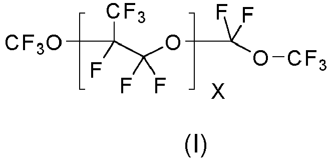 wobei x eine Ganzzahl von 10 bis 60 ist.

6. Die nicht-therapeutische Verwendung von einem Perfluoropolyether nach Anspruch 5, wobei x eine Anzahl von 20 bis 40 ist.

7. Die nicht-therapeutische Verwendung von einem Perfluoropolyether nach einem der Ansprüche 5-6, wobei x = 30 ist.

8. Die nicht-therapeutische Verwendung von einem Perfluoropolyether nach einem der Ansprüche 1-7, wobei die Lichttherapiebehandlung eine Lasertherapiebehandlung ist.

9. Die nicht-therapeutische Verwendung von einem Perfluoropolyether nach einem der Ansprüche 1-8, wobei die topische Zusammensetzung eine wirksame Menge des Perfluoropolyethers umfasst, die von 0,1 Gew.-% bis 10 Gew.-% bezogen auf das gesamte Gewicht der Zusammensetzung reicht.

10. Die nicht-therapeutische Verwendung von einem Perfluoropolyether nach einem der Ansprüche 1-9, wobei die topische Zusammensetzung eine wirksame Menge des Perfluoropolyethers umfasst, die von 1,5 Gew.-% bis 5 Gew.-% bezogen auf das gesamte Gewicht der Zusammensetzung reicht.

11. Die nicht-therapeutische Verwendung von einem Perfluoropolyether nach einem der Ansprüche 1-10, wobei die topische Zusammensetzung folgendes umfasst:
von 0,1% bis 10%, bezogen auf das Gewicht des Perfluoropolyethers;
von 8% bis 15%, bezogen auf das Gewicht von mindestens einem Emolliens;
von 1 bis 9%, bezogen auf das Gewicht von mindestens einem Emulgator;
von 0,01% bis 3%, bezogen auf das Gewicht von mindestens einem Tensid;
von 0,05% bis 1%, bezogen auf das Gewicht von mindestens einem pH-Regulierungsmittel;
von 0,001 bis 0,12%, bezogen auf das Gewicht von mindestens einem Chelator;
von 0,002% bis 0,15%, bezogen auf das Gewicht von mindestens einem Antioxidationsmittel;
von 0% bis 1%, bezogen auf das Gewicht von mindestens einem Konservierungsmittel;
von 0% bis 5% bezogen auf das Gewicht von mindestens einem Hautpflegestoff; und qs (*quantum satis*) von mindestens einem Lösungsmittel,
so dass die Summe der Ingredienzen 100% beträgt.

12. Die nicht-therapeutische Verwendung von einem Perfluoropolyether nach einem der Ansprüche 1-11, wobei die topische Zusammensetzung folgendes umfasst:
von 0,1% bis 5%, bezogen auf das Gewicht des Perfluoropolyethers;
von 8% bis 10%, bezogen auf das Gewicht von mindestens einem Emolliens;
von 1% bis 8%, bezogen auf das Gewicht von mindesten einem Emulgator;
von 0,01% bis 0,05%, bezogen auf das Gewicht von mindestens einem Tensid;
von 0,05 bis 0,95%, bezogen auf das Gewicht von mindestens einem Konservierungsmittel;
von 0,001% bis 0,5%, bezogen auf das Gewicht von mindestens einem pH-Regulierungsmittel;
von 0,002% bis 0,1%, bezogen auf das Gewicht von mindestens einem Chelator;
von 0,003% bis 0,03%, bezogen auf das Gewicht von mindestens einem Antioxidationsmittel;
von 0,003% bis 1%, bezogen auf das Gewicht von mindesten einem Hautpflegestoff; und
qs von mindestens einem Lösungsmittel, so dass die Summe der Ingredienzen 100% beträgt.

13. Die nicht-therapeutische Verwendung von einem Perfluoropolyether nach einem der Ansprüche 1-12, wobei die topische Zusammensetzung folgendes umfasst:
ca. 1,5%, bezogen auf das Gewicht des Perfluoropolyethers;
ca. 10%, bezogen auf das Gewicht von mindestens einem Emolliens;
ca. 7%, bezogen auf das Gewicht von mindesten einem Emulgator;
ca. 0,04%, bezogen auf das Gewicht von mindestens einem Tensid;
ca. 0,015%, bezogen auf das Gewicht von mindestens einem Antioxidationsmittel;
ca. 0,2%, bezogen auf das Gewicht von mindesten einem pH-Regulierungsmittel;
ca. 0,1%, bezogen auf das Gewicht von mindestens einem Chelator;
ca. 1%, bezogen auf das Gewicht von mindestens einem Konservierungsmittel;
ca. 0,005%, bezogen auf das Gewicht von mindesten einem Hautpflegestoff; und
qs von mindestens einem Lösungsmittel, so dass die Summe der Ingredienzen 100% beträgt.

## Revendications

1. Une utilisation non thérapeutique d'un perfluoropolyéther dans un traitement de luminothérapie d'une condition de la peau, dans laquelle la condition de la peau est de la peau tatouée, dans laquelle le traitement de luminothérapie comprend :
(a) premièrement, appliquer une composition topique comprenant une quantité efficace du perfluoropolyéther conjointement avec un ou plus excipients ou véhicules topiquement acceptables à la région de traitement de la peau ; et, alors
(b) exposer la région de traitement de la peau de l'étape (a) à une ou plus sorties de lumière ;

2. L'utilisation non thérapeutique d'un perfluoropolyéther selon la revendication 1, dans laquelle le nombre de sorties de lumière est compris de 1 à 5.

3. L'utilisation non thérapeutique d'un perfluoropolyéther selon l'une quelconque des revendications 1-2, dans laquelle, lorsque le nombre de sorties est compris de 2 à 5, alors l'intervalle entre les sorties de lumière est égal ou inférieur à 10 minutes.

4. L'utilisation non thérapeutique d'un perfluoropolyéther selon l'une quelconque des revendications 1-3, dans laquelle l'application de perfluoropolyéther de l'étape (a) est effectuée sans occlusion de la peau.

5. L'utilisation non thérapeutique d'un perfluoropolyéther selon l'une quelconque des revendications 1-4, dans laquelle le perfluoropolyéther est un composé de formule (I) 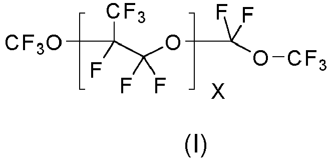 dans laquelle x est un nombre entier de 10 à 60.

6. L'utilisation non thérapeutique d'un perfluoropolyéther selon la revendication 5, dans laquelle x est un nombre entier allant de 20 à 40.

7. L'utilisation non thérapeutique d'un perfluoropolyéther selon l'une quelconque des revendications 5-6, dans laquelle x est 30.

8. L'utilisation non thérapeutique d'un perfluoropolyéther selon l'une quelconque des revendications 1-7, dans laquelle le traitement de luminothérapie est un traitement de thérapie au laser.

9. L'utilisation non thérapeutique d'un perfluoropolyéther selon l'une quelconque des revendications 1-8, dans laquelle la composition topique comprend une quantité efficace du perfluoropolyéther comprise de 0,1 % à 10 % en poids par rapport au poids total de la composition.

10. L'utilisation non thérapeutique d'un perfluoropolyéther selon l'une quelconque des revendications 1-9, dans laquelle la composition topique comprend une quantité efficace du perfluoropolyéther comprise de 1,5 % à 5 % en poids par rapport au poids total de la composition.

11. L'utilisation non thérapeutique d'un perfluoropolyéther selon l'une quelconque des revendications 1-10, dans laquelle la composition topique comprend :
de 0,1 % à 10 % en poids du perfluoropolyéther ;
de 8 % à 15 % en poids d'au moins un émollient ;
de 1 % à 9 % en poids d'au moins un agent émulsifiant ;
de 0,01 % à 3 % en poids d'au moins un tensioactif ;
de 0,05 % à 1 % en poids d'au moins un agent de régulation du pH ;
de 0,001 à 0,12 % en poids d'au moins un chélateur ;
de 0,002 % à 0,15 % en poids d'au moins un antioxydant ;
de 0 % à 1 % en poids d'au moins un conservateur ;
de 0 % à 5 % en poids d'au moins un agent de conditionnement de la peau ;
et la quantité suffisante d'au moins un solvant de manière que la somme des ingrédients soit 100 %.

12. L'utilisation non thérapeutique d'un perfluoropolyéther selon l'une quelconque des revendications 1-11, dans laquelle la composition topique comprend :
de 0,1 % à 5% en poids du perfluoropolyéther ;
de 8 % à 10% en poids d'au moins un émollient ;
de 1 % à 8% en poids d'au moins un agent émulsifiant ;
de 0,01 % à 0,05 % en poids d'au moins un tensioactif ;
de 0,05 à 0,95 % en poids d'au moins un conservateur ;
de 0,001 % à 0,5 % en poids d'au moins un agent de régulation du pH ;
de 0,002 % à 0,1 % en poids d'au moins un chélateur ;
de 0,003 % à 0,03 % en poids d'au moins un antioxydant ;
de 0,003 % à 1 % en poids d'au moins un agent de conditionnement de la peau ; et
la quantité suffisante d'au moins un solvant de manière que la somme des ingrédients soit 100 %.

13. L'utilisation non thérapeutique d'un perfluoropolyéther selon l'une quelconque des revendications 1-12, dans laquelle la composition topique comprend :
environ 1,5 % en poids du perfluoropolyéther ;
environ 10 % en poids d'au moins un émollient ;
environ 7 % en poids d'au moins un agent émulsifiant ;
environ 0,04 % en poids d'au moins un tensioactif ;
environ 0,015 % en poids d'au moins un antioxydant ;
environ 0,2 % en poids d'au moins un agent de régulation du pH ;
environ 0,1 % en poids d'au moins un chélateur ;
environ 1 % en poids d'au moins un conservateur ;
environ 0,005 % en poids d'au moins un agent de conditionnement de la peau ; et
la quantité suffisante d'au moins un solvant de manière que la somme des ingrédients soit 100 %.
